# EUROPEAN PATENT SPECIFICATION

(11) **EP 4 520 372 B1**
(45) Date of publication and mention of the grant of the patent: **05.08.2026**
(21) Application number: 23868313.0
(22) Date of filing: 02.06.2023
(51) Int. Cl.: A61M 15/00, A61M 15/06

(54) **INHALER**
INHALATOR
INHALATEUR

(30) Priority: 19.09.2022 KR 20220117659
(43) Date of publication of application: 12.03.2025
(73) Proprietor: KT&G Corporation, Daedeok-gu Daejeon 34337 (KR)
(72) Inventor: JUNG, Yongmi, Daejeon 34128 (KR); KIM, Moonwon, Daejeon 34128 (KR); SHIN, Jun Won, Daejeon 34128 (KR)
(74) Representative: Ter Meer Steinmeister & Partner
(86) International application number: PCT/KR2023/007610
(87) International publication number: WO 2024/063250

(56) References cited:
- EP-A1- 1 245 243
- EP-A1- 4 483 929
- EP-A1- 4 520 370
- WO-A1-01/26720
- WO-A1-2016/156212
- WO-A1-2019/082057
- WO-A1-2020/214593
- WO-A1-2021/079345
- WO-A2-91/19524
- CN-A- 110 812 635
- DE-U1- 202021 003 743
- JP-A- 2008 520 592
- KR-A- 20190 095 290
- US-A- 3 888 252
- US-A1- 2003 000 523
- US-A1- 2012 255 546
- US-A1- 2014 305 454
- US-B2- 10 173 019
- US-B2- 7 722 566

## Description

### TECHNICAL FIELD

The following embodiments relate to an inhaler.

### BACKGROUND ART

Research has been conducted on an inhaler that may deliver a target substance directly to the lungs of a user. For example, Korean Patent Publication No. 10-2016-0065204 discloses a dry powder inhaler.

EP 4 483 929 A1 describes an inhaler comprising a first holder, a second holder rotatably connected thereto, a piercing member, and a functional substance accommodation member that is opened by the piercing member upon rotation of the second holder.

EP 4 520 370 A1 describes an inhaler comprising a holder with an opening and inner space, a piercing member protruding toward the opening and having a plurality of air-permeable holes, and a functional material accommodation member inserted into the holder to contain the functional material.

WO 2019/082057 A1 describes an inhaler system comprising an inhaler article and a piercing article with a recessed piercing element that pierces a single hole into a capsule contained in the inhaler article when the inhaler article is inserted.

WO 2021/079345 A1 describes a holder for an inhaler article comprising a housing with a cavity and a movable sleeve configured to retain the inhaler article, the sleeve having an air inlet arranged to induce a swirl in the air entering the sleeve cavity.

US 2014/305454 A1 describes a vaporizer device with a mouthpiece, a dual-coil or sinuous thermal resistor allowing fluid flow, and a permeable membrane in contact with the resistor.

US 3 888 252 A describes a device for inhaling medicinal powders, comprising a tube with a desiccator and balloon, a mouthpiece with a capsule retainer, where assembling perforates the capsule to release powder into the airflow for inhalation.

US 10 173 019 B2 describes a method of delivering medicament powder by directing a gas through or near the powder, releasing a therapeutically effective amount, optionally based on breath or time, with the powder held in a capsule having apertures.

### DISCLOSURE OF THE INVENTION

### TECHNICAL GOALS

An inhaler according to various embodiments may induce a vortex behavior of functional substance powder.

An inhaler according to various embodiments may induce smooth discharge of functional substance powder.

According to the invention, there is provided an inhaler according to the independent claim. Preferred embodiments are given in the dependent claims.

### TECHNICAL SOLUTIONS

An inhaler according to the invention comprises a holder including an opening and an internal space that communicates with the opening, a piercing member protruding toward the internal space, and a functional substance accommodation member inserted from the opening into the internal space of the holder along a longitudinal axis of the holder and configured to accommodate the functional substance, wherein one end of the piercing member is fixed to one side of the holder, and a cutting edge of the piercing member is directed toward the longitudinal axis.

According to the invention, the piercing member includes a first piercing member and a second piercing member disposed spaced apart from the first piercing member along the longitudinal axis.

According to an embodiment, the first piercing member and the second piercing member may be disposed to face each other about the longitudinal axis.

According to an embodiment, a distance in a horizontal direction, which may be perpendicular to the longitudinal axis, between a cutting edge of the first piercing member and a cutting edge of the second piercing member may be less than a horizontal width of the functional substance accommodation member.

According to the invention, the functional substance accommodation member includes a stick configured to be insertable into the internal space of the holder and a capsule disposed in an inside of the stick and configured to accommodate the functional substance.

According to the invention, a marker for indicating a degree of insertion of the stick into the internal space is provided in at least one of the stick or the holder.

According to an embodiment, the marker may indicate a first insertion state and a second insertion state of the stick, the first insertion state may be a position at which the capsule starts contacting the piercing member, and the second insertion state may be a position at which the stick reaches an end of the internal space.

According to an embodiment, the marker may indicate a third insertion state of the stick, and the third insertion state may be a state in which the stick is inserted into a position between the first insertion state and the second insertion state.

### EFFECTS OF THE INVENTION

According to various embodiments, a vortex behavior of functional substance powder may be induced.

According to various embodiments, smooth discharge of functional substance powder may be performed by internal airflow introduced into an inhaler penetrating a center of a functional substance accommodation member.

The effects of an inhaler according to various embodiments are not limited to the above-mentioned effects, and other unmentioned effects can be clearly understood from the following description by one of ordinary skill in the art to which the present disclosure pertains.

### Brief Description of Drawings

FIG. 1 is a diagram illustrating an inhaler according to an embodiment of the invention.
FIG. 2 is a diagram illustrating an operating state of an inhaler according to an embodiment of the invention.
FIG. 3 is a diagram illustrating a puff state of an inhaler according to an embodiment of the invention.

### BEST MODE FOR CARRYING OUT THE INVENTION

The terms used in the embodiments are selected from among common terms that are currently widely used, in consideration of their function in the embodiments. However, the terms may become different according to an intention of one of ordinary skill in the art, a precedent, or the advent of new technology. Also, in particular cases, the terms are discretionally selected by the applicant of the disclosure, and the meaning of those terms will be described in detail in the corresponding part of the detailed description. Therefore, the terms used in the disclosure are not merely designations of the terms, but the terms are defined based on the meaning of the terms and content throughout the disclosure.

It will be understood that when a certain part "includes" a certain component, the part does not exclude another component but may further include another component, unless the context clearly dictates otherwise. Also, terms such as "unit," "module," etc., as used in the specification may refer to a part for processing at least one function or operation and may be implemented as hardware, software, or a combination of hardware and software.

As used herein, an expression such as "at least one of" that precedes listed components modifies not each of the listed components but all the components. For example, expressions "at least one of a, b, or c" and "at least one of a, b, and c" should be construed as referring to "a," "b," "c," "a and b," "a and c," "b and c," or "a, b, and c."

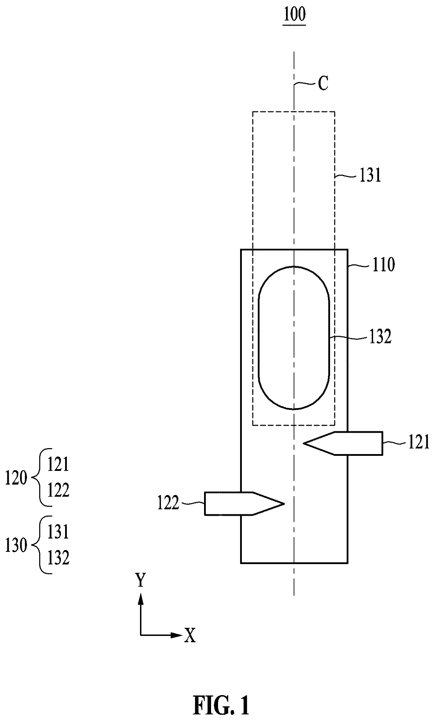

FIG. 1 is a diagram illustrating an inhaler 100 according to an embodiment of the invention.

The inhaler 100 is configured to deliver a functional substance to a user. For example, the inhaler 100 according to an embodiment may be composed of an inhaler that delivers a dried pharmacological substance or nicotine to the lungs of a user in a form of an aerosol.

Referring to FIG. 1, the inhaler 100 includes a holder 110, a piercing member 120, and a functional substance accommodation member 130.

The holder 110 may be configured in a cylindrical shape and has an internal space, and the internal space is formed to a size into which the functional substance accommodation member 130, which is described below, is inserted. An opening is formed in a side of the holder 110, wherein the functional substance accommodation member 130 is inserted through the opening, and the opening and the internal space are connected to each other.

The piercing member 120 includes a first piercing member 121 and a second piercing member 122. One side of the first piercing member 121 is fixed to an inside of the holder 110, and the other side of the first piercing member 121 is configured as a cutting edge protruding toward a longitudinal axis C of the holder 110. One side of the second piercing member 122 is fixed to the inside of the holder 110, and the other side of the second piercing member 122 is configured as a cutting edge protruding toward the longitudinal axis C of the holder 110.

The first piercing member 121 and the second piercing member 122 are disposed spaced apart in a direction parallel to the longitudinal axis C. In particular, the first piercing member 121 and the second piercing member 122 are disposed spaced apart from each other in a ±Y direction of FIG. 1.

In addition, the first piercing member 121 and the second piercing member 122 may be disposed to face each other about the longitudinal axis C. For example, the first piercing member 121 and the second piercing member 122 may be disposed to be spaced apart from each other in a ±X direction of FIG. 1 with respect to the longitudinal axis C.

The functional substance accommodation member 130 includes a stick 131 and a capsule 132. The stick 131 may be configured in a cylindrical shape and is inserted into the internal space of the holder 110, and the capsule 132 is disposed in an inside of the stick 131.

An area of the functional substance accommodation member 130, in which the capsule 132 may be disposed, may be set so that the capsule 132 may be at a position overlapping the first piercing member 121 or the second piercing member 122 when the stick 131 reaches an end of the internal space of the holder 110.

The capsule 132 accommodates a functional substance. The functional substance may include, for example, at least one of nicotine, theanine, caffeine, taurine, a pharmacological substance, or a mixture thereof. The functional substance may be in a form of fine granules or dry powder.

In an example, an outer shell of the capsule 132 may be made of a material that may be pierced or cut by the first piercing member 121 or the second piercing member 122.

In an embodiment, the first piercing member 121 and the second piercing member 122 may be fixed to the holder 110 at positions at which a horizontal distance (e.g., a distance in a ±X direction of FIG. 1) between the first piercing member 121 and the second piercing member 122 is less than an outer diameter of the capsule 132. Here, a horizontal direction is a direction perpendicular to the longitudinal axis C.

Since a horizontal distance between the cutting edge of the first piercing member 121 and the cutting edge of the second piercing member 122 may be less than a horizontal width of the capsule 132, when the stick 131 is inserted into the internal space of the holder 110, at least a portion of the capsule 132 may be cut by the first piercing member 121 or the second piercing member 122 and thus the functional substance in the capsule 132 may be exposed to an outside of the capsule 132

In an embodiment, a A marker (not shown) for indicating a degree of insertion of the stick 131 into the internal space is provided in at least one of the holder 110 or the stick 131 of the functional substance accommodation member 130. When an open area in a longitudinal slit shape is formed in the capsule 132, a size of the slit may be indirectly measured by the marker, and the size of the slit may be set to be proportional to an amount of a puff of the functional substance.

In an embodiment, the marker may indicate a first insertion state and a second insertion state of the stick 131. The first insertion state may be a position at which the capsule 132 starts contacting the piercing member 120, and the second insertion state may be a position at which the stick 131 reaches the end of the internal space.

In addition, the marker may indicate a third insertion state of the stick 131, and the third insertion state may be a state in which the stick 131 is inserted into a position between the first insertion state and the second insertion state.

For example, as the stick 131 is inserted into the internal space of the holder 110 and moves toward the end of the internal space, an end of the stick 131 may be the first to be cut by the first piercing member 121 or the second piercing member 122. Here, it may be confirmed that the first piercing member 121 may start cutting the capsule 132 when the stick 131 reaches the first insertion state indicated on the marker. The user may adjust a desired amount of a puff by further moving the stick 131 toward the third insertion state indicated on the marker. Alternatively, the user may set the desired amount of a puff to a maximum amount of a puff by moving the stick 131 toward the second insertion state indicated on the marker. For example, according to the number of puffs, the amount of a puff of the stick 131 may be adjusted to maintain the third insertion state at an initial puff and maintain the second insertion state at subsequent puffs.

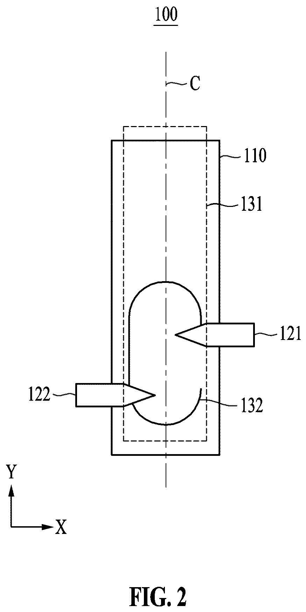

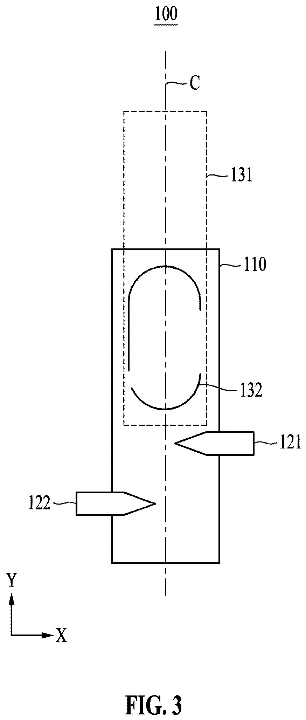

FIG. 2 and FIG. 3 are diagrams illustrating an operating state of the inhaler 100 according to an embodiment of the invention. FIG. 2 illustrates a state in which the functional substance accommodation member 130 is inserted into the internal space of the holder 110, and FIG. 3 illustrates a state in which the functional substance may transfer while a portion of the capsule 132 is cut.

Referring to FIG. 2, the functional substance accommodation member 130 is inserted into the internal space of the holder 110. Here, at least a portion of the capsule 132 may be cut by the first piercing member 121 or the second piercing member 122 and thus a longitudinal slit may be formed in the capsule 132.

As described above, the stick 131 may be inserted into the internal space to one of the second insertion state or the third insertion state by the marker and thus a degree of opening of the slit may be adjusted.

A slit-shaped open area by the first piercing member 121 and a slit-shaped open area by the second piercing member 122 may be formed in the capsule 132. Since the open areas may be formed spaced apart along the longitudinal axis C, airflow passing through the open areas may pass through a central area of the capsule 132.

Referring to FIG. 3, at least a portion of the functional substance accommodation member 130 may be separated from the holder 110. Here, the functional substance accommodation member 130 may be completely separated from the holder 110, but the functional substance may transfer while the capsule 132 is inserted in the holder 110. In this case, separation of the functional substance may effectively be prevented compared to a case in which the functional substance accommodation member 130 is completely separated from the holder 110, and thus, unnecessary leakage of the functional substance may be prevented.

When the user bites one end of the stick 131 and makes an inhaling motion while the open area is formed in the capsule 132, external air may flow into the internal space of the holder 110 through an airflow hole (not shown) formed in the holder 110, and the air may flow into the capsule 132 through the open area formed by the second piercing member 122. Subsequently, the air may escape through the open area formed by the first piercing member 121 and reach the user, in which case the functional substance in the capsule 132 may also be transferred to the user. Due to eccentric positions of the open areas, the air may pass through a central part of the capsule 132 and, at the same time, a vortex behavior of the functional substance may be induced. The vortex behavior may induce smooth movement of the functional substance, and accordingly, discharge of the functional substance may be performed more smoothly.

In an example, when the capsule 132 is disposed in the stick 131 in an unfixed state, air movement (airflow) generated due to an eccentric position of the open area may induce the rotation of the capsule 132. Through this, the discharge of the functional substance may be performed more smoothly.

## Claims

1. An inhaler (100) for inhaling a functional substance, the inhaler (100) comprising:
a holder (110) comprising an opening and an internal space that communicates with the opening;
a piercing member (120; 121, 122) protruding toward the internal space; and
a functional substance accommodation member (130) inserted from the opening into the internal space of the holder (110) along a longitudinal axis (C) of the holder (110) and configured to accommodate the functional substance,
the functional substance accommodation member (130) comprising a stick (131) configured to be insertable into the internal space of the holder (110) and a capsule (132) disposed in an inside of the stick (131) and configured to accommodate the functional substance,
wherein one end of the piercing member (120; 121, 122) is fixed to one side of the holder (110), and a cutting edge of the piercing member (120; 121, 122) is directed toward the longitudinal axis (C),
wherein the piercing member (120;121, 122) comprises a first piercing member (121) and a second piercing member (122) disposed spaced apart from the first piercing member (121) along the longitudinal axis (C), and
wherein a marker for indicating a degree of insertion of the stick into the internal space is provided in at least one of the stick or the holder (110).

2. The inhaler (100) of claim 1, wherein the first piercing member (121) and the second piercing member (122) are disposed to face each other about the longitudinal axis (C).

3. The inhaler (100) of claim 1, wherein a distance in a horizontal direction, which is perpendicular to the longitudinal axis (C), between a cutting edge of the first piercing member (121) and a cutting edge of the second piercing member (122) is less than a horizontal width of the functional substance accommodation member (130).

4. The inhaler (100) of claim 1, wherein the marker indicates a first insertion state and a second insertion state of the stick (131), and
wherein the first insertion state is a position at which the capsule (132) starts contacting the piercing member (120; 121, 122), and the second insertion state is a position at which the stick (131) reaches an end of the internal space.

5. The inhaler (100) of claim 4, wherein the marker indicates a third insertion state of the stick (131), and
wherein the third insertion state is a state in which the stick (131) is inserted into a position between the first insertion state and the second insertion state.

## Patentansprüche

1. Inhalationsvorrichtung (100) zum Inhalieren einer funktionalen Substanz, wobei die Inhalationsvorrichtung (100) Folgendes umfasst:
einen Halter (110), der eine Öffnung und einen Innenraum, der mit der Öffnung in Verbindung steht, umfasst;
ein Durchstoßelement (120; 121, 122), das in Richtung des Innenraums vorsteht; und
ein Aufnahmeelement (130) für eine funktionale Substanz, das durch die Öffnung entlang einer Längsachse (C) des Halters (110) in den Innenraum des Halters (110) eingesetzt wird und konfiguriert ist, die funktionale Substanz aufzunehmen,
wobei das Aufnahmeelement (130) für eine funktionale Substanz einen Stift (131), der konfiguriert ist, in den Innenraum des Halters (110) eingesetzt werden zu können, und eine Kapsel (132), die in einem Innenraum des Stifts (131) angeordnet ist und konfiguriert ist, die funktionale Substanz aufzunehmen, umfasst,
wobei ein Ende des Durchstoßelements (120; 121, 122) an einer Seite des Halters (110) befestigt ist und eine Schneidkante des Durchstoßelements (120; 121, 122) in Richtung der Längsachse (C) ausgerichtet ist,
wobei das Durchstoßelement (120; 121, 122) ein erstes Durchstoßelement (121) und ein zweites Durchstoßelement (122), das vom ersten Durchstoßelement (121) entlang der Längsachse (C) beabstandet angeordnet ist, umfasst, und
wobei eine Markierung zum Angeben eines Grads des Einsetzens des Stifts in den Innenraum im Stift und/oder im Halter (110) vorgesehen ist.

2. Inhalationsvorrichtung (100) nach Anspruch 1, wobei das erste Durchstoßelement (121) und das zweite Durchstoßelement (122) derart angeordnet sind, dass sie einander über die Längsachse (C) zugewandt sind.

3. Inhalationsvorrichtung (100) nach Anspruch 1, wobei ein Abstand in einer horizontalen Richtung, die zur Längsachse (C) senkrecht ist, zwischen einer Schneidkante des ersten Durchstoßelements (121) und einer Schneidkante des zweiten Durchstoßelements (122) kleiner als eine horizontale Breite des Aufnahmeelements (130) für eine funktionale Substanz ist.

4. Inhalationsvorrichtung (100) nach Anspruch 1, wobei die Markierung einen ersten Einsetzzustand und einen zweiten Einsetzzustand des Stifts (131) angibt und
wobei der erste Einsetzzustand eine Position ist, an der die Kapsel (132) beginnt, das Durchstoßelement (120; 121, 122) zu berühren, und der zweite Einsetzzustand eine Position ist, an der der Stift (131) ein Ende des Innenraums erreicht.

5. Inhalationsvorrichtung (100) nach Anspruch 4, wobei die Markierung einen dritten Einsetzzustand des Stifts (131) angibt und
wobei der dritte Einsetzzustand ein Zustand ist, in dem der Stift (131) an einer Position zwischen dem ersten Einsetzzustand und dem zweiten Einsetzzustand eingesetzt ist.

## Revendications

1. Inhalateur (100) destiné à inhaler une substance fonctionnelle, l'inhalateur (100) comportant :
un support (110) comportant une ouverture et un espace interne qui communique avec l'ouverture ;
un élément de percement (120 ; 121, 122) faisant saillie vers l'espace interne ; et
un élément de réception de substance fonctionnelle (130) inséré à partir de l'ouverture dans l'espace interne du support (110) le long d'un axe longitudinal (C) du support (110) et configuré pour recevoir la substance fonctionnelle,
l'élément de réception de substance fonctionnelle (130) comportant un bâtonnet (131) configuré pour pouvoir être inséré dans l'espace interne du support (110) et une capsule (132) disposée dans un intérieur du bâtonnet (131) et configurée pour recevoir la substance fonctionnelle,
dans lequel une extrémité de l'élément de percement (120 ; 121, 122) est fixée à un côté du support (110), et un bord tranchant de l'élément de percement (120 ; 121, 122) est dirigé vers l'axe longitudinal (C),
dans lequel l'élément de percement (120 ; 121, 122) comporte un premier élément de percement (121) et un second élément de percement (122) disposés espacés du premier élément de percement (121) le long de l'axe longitudinal (C), et
dans lequel un marqueur destiné à indiquer un degré d'insertion du bâtonnet dans l'espace interne est prévu dans au moins un élément parmi le bâtonnet ou le support (110).

2. Inhalateur (100) selon la revendication 1, dans lequel le premier élément de percement (121) et le second élément de percement (122) sont disposés de manière à se faire face autour de l'axe longitudinal (C).

3. Inhalateur (100) selon la revendication 1, dans lequel une distance dans une direction horizontale, qui est perpendiculaire à l'axe longitudinal (C), entre un bord tranchant du premier élément de percement (121) et un bord tranchant du second élément de percement (122) est inférieure à une largeur horizontale de l'élément de réception de substance fonctionnelle (130).

4. Inhalateur (100) selon la revendication 1, dans lequel le marqueur indique un premier état d'insertion et un deuxième état d'insertion du bâtonnet (131), et
dans lequel le premier état d'insertion est une position à laquelle la capsule (132) commence à être en contact avec l'élément de percement (120 ; 121, 122), et le deuxième état d'insertion est une position à laquelle le bâtonnet (131) atteint une extrémité de l'espace interne.

5. Inhalateur (100) selon la revendication 4, dans lequel le marqueur indique un troisième état d'insertion du bâtonnet (131), et
dans lequel le troisième état d'insertion est un état dans lequel le bâtonnet (131) est inséré dans une position située entre le premier état d'insertion et le deuxième état d'insertion.
